# FASCICULE DE BREVET EUROPEEN

(11) **EP 1 685 146 B1**
(45) Date de publication et mention de la délivrance du brevet: **25.04.2007**
(21) Numéro de dépôt: 04787389.8
(22) Date de dépôt: 16.09.2004
(51) Int. Cl.: C07H 17/08

(54) **COMPOSE CRISTALLIN SOUS FORME LIBRE OU DE SOLVATE AVEC L'ETHANOL DE 4 -DEMETHYL-4' -PHOSPHATE-2",3"-BISPENTA FLUOROPHENOXYACETYL-4",6"-ETHYLIDENE-ß-D-GLUCOSIDE D' EPIPODOPHYLLOTOXINE**
KRISTALLINE VERBINDUNG VON 4'-DEMETHYL-4'-PHOSPHAT-2'',3''-BISPENTAFLUORPHENOXYACETYL-4'',6''-ETHYLIDEN-ß-D-EPIPODOPHYLLOTOXINGLUKOSID ENTWEDER IN DER FREIEN FORM ODER SOLVATISIERT MIT ETHANOL
CRYSTALLINE COMPOUND OF 4'-DEMETHYL-4'-PHOSPHATE-2",3"-BISPENTAFLUOROPHENOXYACETYL-4",6"-ETHYLIDENE-ß-D-EPIPODOPHYLLOTOXIN GLUCOSIDE EITHER IN ITS FREE FORM OR SOLVATED WITH ETHANOL

(30) Priorité: 16.09.2003 FR 0310836
(43) Date de publication de la demande: 02.08.2006
(73) Titulaire: PIERRE FABRE MEDICAMENT, 92100 Boulogne (FR)
(72) Inventeur: GUMINSKI, Yves, F-81090 Lagarrigue (FR); IMBERT, Thierry, F-81290 Viviers-les-Montagnes (FR); KRUCZYNSKI, Anna, 31450 Pompertuzat (FR)
(74) Mandataire: Ahner, Francis
(86) Numéro de dépôt international: PCT/FR2004/002349
(87) Numéro de publication internationale: WO 2005/028492

(56) Documents cités:
- WO-A-00/58324
- WO-A-96/12727

## Description

Le 4'-déméthyl-4'-phosphate-2",3 "-bis pentafluorophénoxyacétyl-4",6"-éthylidène-β-D-glucoside d'épipodophyllotoxine sous forme de di sel de N-méthyl-D-glucamine, selon la formule 1, est connu par sa structure, sa préparation et son activité anticancéreuse (WO 96/12727). Un autre procédé de préparation de ce composé est également décrit dans le brevet FR 2 791 682.

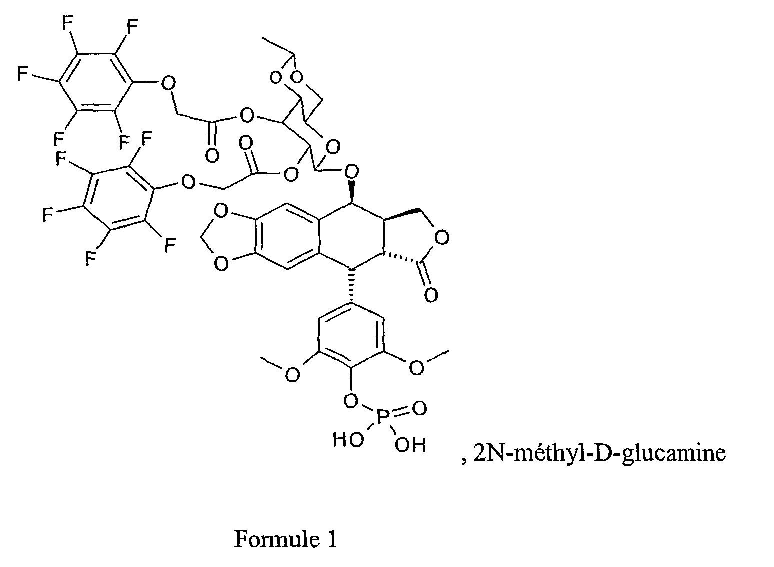

Ce composé de formule 1 possède une activité anticancéreuse particulièrement importante. Son activité anti-tumorale a été mise en évidence in vivo. En particulier il permet une longue survie à la souris sur laquelle a été implanté le modèle de la leucémie P388 *(British Journal of Cancer* (2000), 83(11), 1516-1524). De plus la puissance de l'inhibition de l'activité in vitro des topoisomérases 1 et 2 a été montrée avec ce composé *(Biochem. Pharmacol.* (2000), 59, 807).
Ce composé 1 est stable dans les conditions usuelles de pH neutre et acide et de température. Mais si l'on veut le conserver sur une longue durée, il est nécessaire de le conserver à l'abri de l'humidité dans un flacon hermétique et à -20°C. Par contre s'il est conservé à 4°C et à fortiori à température ambiante, ce composé se dégrade avec le temps. Il ne peut donc pas être manipulé de façon satisfaisante, pour servir dans un protocole de traitement anticancéreux en milieu hospitalier. Il doit donc être préparé au moment de l'emploi, ce qui est un handicap.
Le précurseur chimique de ce composé est le dérivé phosphate libre, de formule 2 : 4'-déméthyl-4'-phosphate-2",3 "-bispentafluorophénoxyacétyl-4",6"éthylidène-β-D-glucoside d'épipodophyllotoxine.

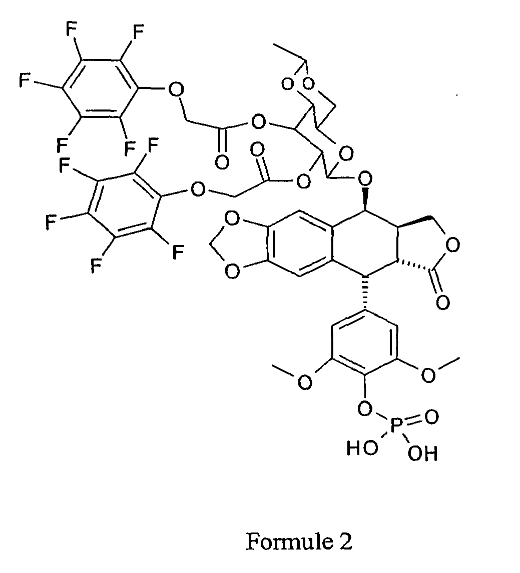

Ce composé n'est pas isolé et est utilisé directement sous forme amorphe dans la préparation de son sel de N-méthyl-D-glucamine, de formule 1 (WO 96/12727). Ce dérivé phosphate libre de formule 2, une fois isolé, se présente sous une forme amorphe hygroscopique et peu stable. Il est nécessaire alors de former le sel N-méthyl-D-glucamine rapidement, dans des conditions anhydres et de le conserver au froid, ou de l'utiliser rapidement. Ce dérivé de formule 2 sous forme amorphe ne peut donc pas lui-même être stocké et manipulé dans de bonnes conditions au niveau industriel. De plus, il a été observé que le composé de formule 2, sous forme amorphe, ne présente pas les propriétés biologiques importantes du composé de formule 1. Un problème reste donc à résoudre, celui d'obtenir le composé de formule 2 sous forme cristalline.

Il est connu classiquement que la cristallisation d'un composé amorphe peut présenter de très grosses difficultés, et l'obtention des premiers cristaux est toujours problématique.
Ultérieurement, la cristallisation se produit plus facilement par ensemencement.

Un solvate avec 2 molécules d'éthanol a été décrit dans le brevet EP 537555 pour le composé étopofos, qui est la prodrug de l'étoposide. Il était logique d'espérer obtenir une cristallisation du composé de formule 2, en opérant de façon similaire au procédé décrit dans ce brevet.
La méthode d'obtention du solvate, diéthanolate, préconisée dans ce brevet (traitement par l'éthanol avec un cosolvant ou l'eau) ne convient pas à notre cas.
Il existe une différence très importante de solubilité entre l'étopofos et le produit de formule 2.
L'étopofos est soluble dans l'eau (100 mg/ml) alors que le composé de formule 2, amorphe, ne l'est pas. Ce dernier est soluble dans l'éthanol et l'addition d'eau préconisée pour l'obtention du solvate de l'étopofos donne dans notre cas une gomme non cristallisable.
De nombreux essais ont été réalisés au laboratoire pour essayer de cristalliser le composé de formule 2. Des alcools comme le méthanol, l'éthanol, l'alcool isopropylique, le butanol ont été utilisés sans succès, ainsi que d'autres types de solvants comme l'acétate d'éthyle, l'acétone, la méthyléthylcétone, le dioxane. Seul l'emploi d'éthers comme l'éther éthylique ou l'éther isopropylique ou d'alcanes comme le pentane, l'heptane ou l'éther de pétrole fournit un produit poudre solide mais sous forme amorphe.
Il a donc été trouvé de façon surprenante que le phosphate libre de formule 2 possède la propriété de former dans des conditions particulières un solvate avec ½ molécule d'éthanol pour conduire à son dérivé hémiéthanolate cristallisé et stable, de formule 3, ce dérivé donnant après séchage le dérivé de formule 2 sous forme cristalline. De plus, par encemencement à partir du composé cristallin de formule 2 ou 3, il a été possible d'obtenir d'autre solvates cristallins avec l'éthanol que le solvate hémiéthanolate du 4'-déméthyl-4'-phosphate-2",3"-bispentafluorophénoxyacétyl-4",6"-éthylidène-β-D-glucoside d'epipodophyllotoxine.

La présente invention concerne donc le composé cristallin 4'-déméthyl-4'-phosphate-2",3"-bispentafluorophénoxyacétyl-4",6"-éthylidène-β-D-glucoside d'épipodophyllotoxine sous forme libre de formule 2 suivante : 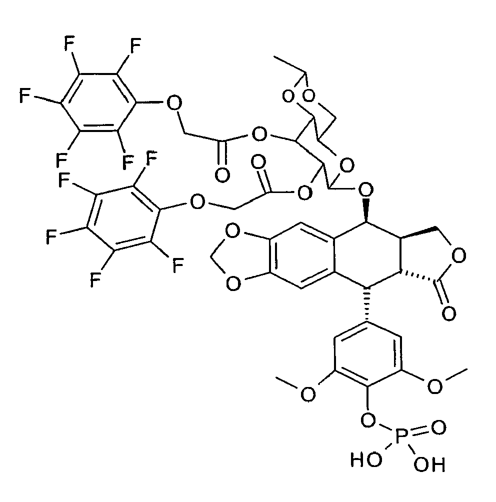 ou sous forme de solvate avec l'éthanol. Avantageusement, le solvate avec l'éthanol est un solvate hémiéthanolate de formule 3 suivante :

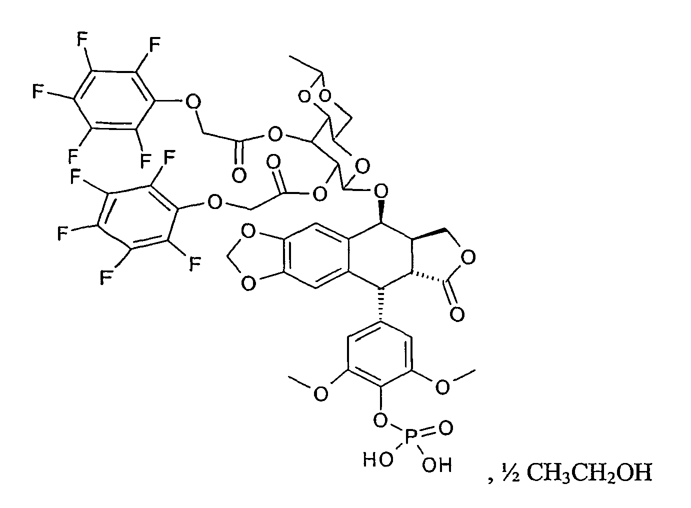

Avantageusement le diagramme de rayon X du composé cristallin sous forme libre de formule 2 correspond sensiblement à celui de la figure 2 et celui du composé cristallin sous forme de solvate hémiéthanolate de formule 3 correspond sensiblement à celui de la figure 3. Avantageusement, le diagramme de rayon X d'un composé cristallin du 4'-déméthyl-4'-phosphate-2",3"-bispentafluorophénoxyacétyl-4",6"-éthylidène-β-D-glucoside d'épipodophyllotoxine sous forme de solvate avec l'éthanol autre que le solvate hémiéthanolate de formule 3 correspond sensiblement à celui de la figure 4.

D'autres objets et avantages de l'invention deviendront apparents pour l'homme du métier à partir de la description détaillée ci-dessous et par le biais de références aux dessins illustratifs suivants.

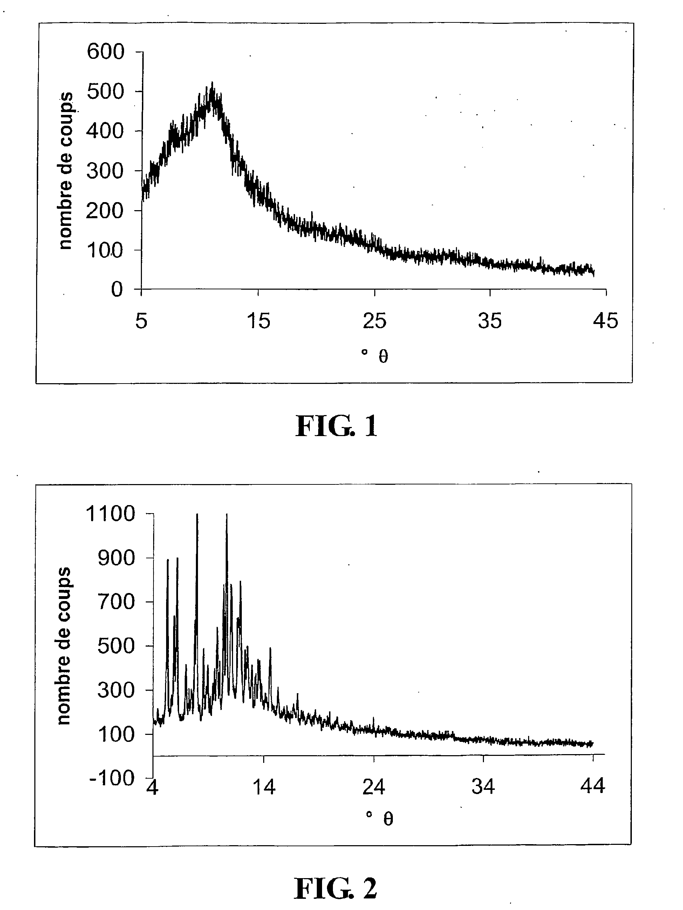

La figure 1 représente le diagramme de rayon X du composé de formule 2 sous forme amorphe

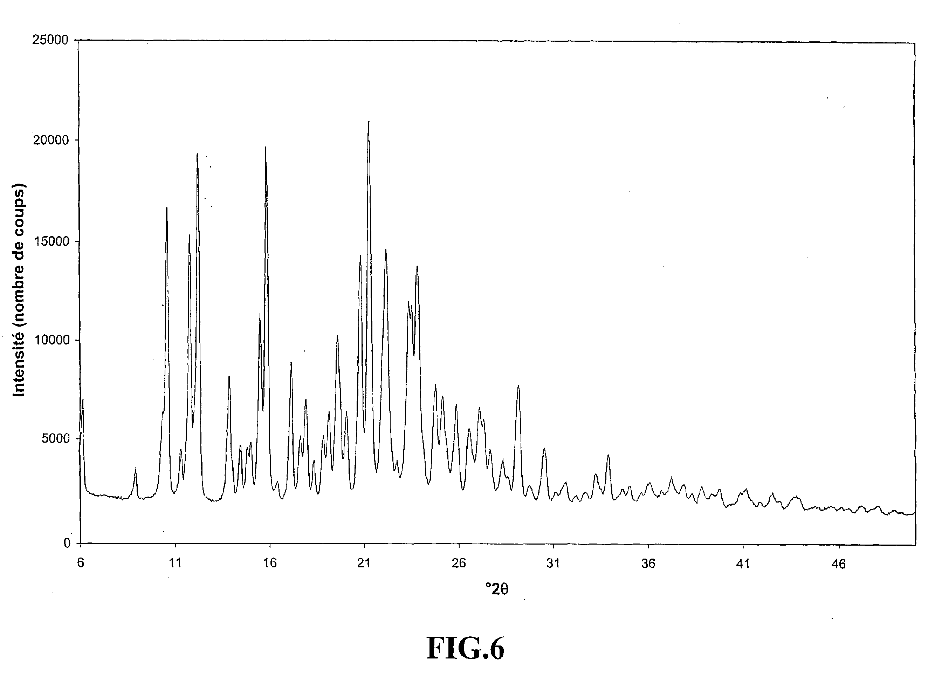

Les figures 2 et 6 représentent le diagramme de rayon X du composé de formule 2 sous forme cristalline

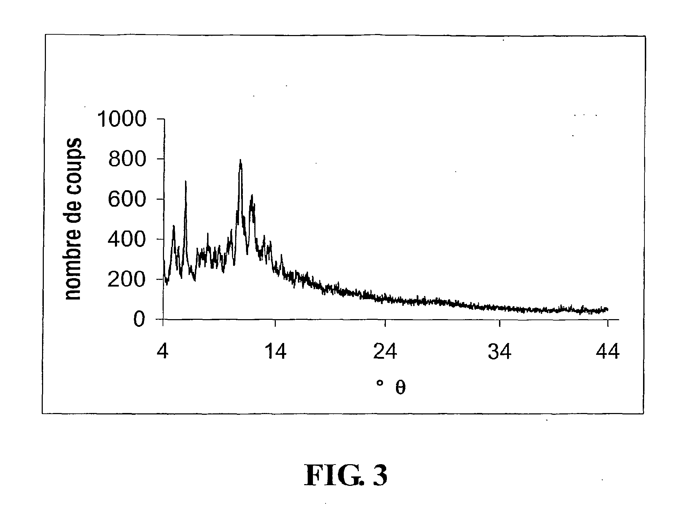

La figure 3 représente le diagramme de rayon X du composé de formule 3 sous forme cristalline

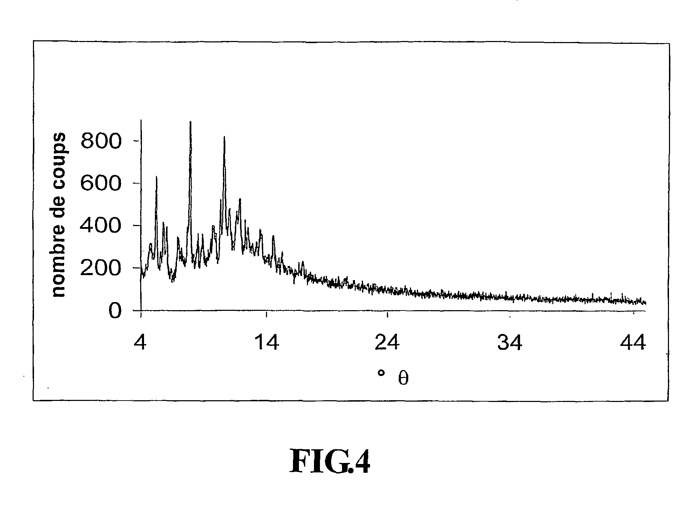

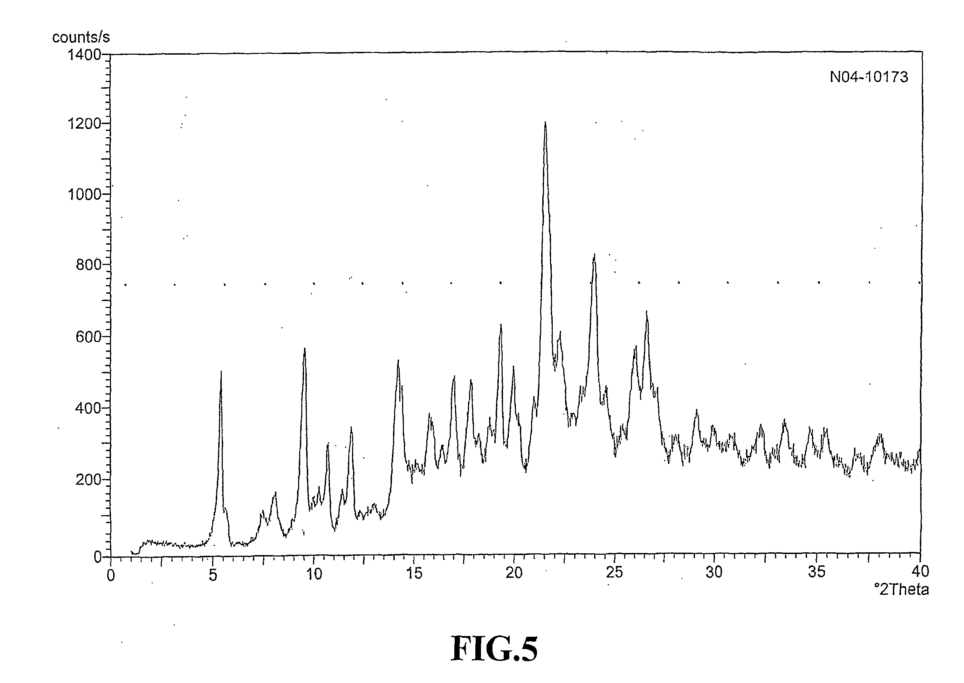

Les figures 4 et 5 représentent le diagramme de rayon X du composé cristallin du 4'-déméthyl-4'-phosphate-2",3"-bispentafluorophénoxyacétyl-4",6"-éthylidène-β-D-glucoside d'épipodophyllotoxine sous forme de solvate avec l'éthanol autre que le solvate hémiéthanolate de formule 3.

La présente invention concerne également un procédé de préparation du composé cristallin sous forme de solvate hémiéthanolate de formule 3 qui comprend l'étape de mise en solution dans l'éthanol anhydre en présence d'ultrasons du composé de formule 2 sous forme amorphe.
Avantageusement, les ultrasons sont appliqués pendant au moins 20 minutes.

Il concerne en outre le procédé de préparation du composé cristallin sous forme libre de formule 2 qui comprend l'étape de séchage du composé cristallin sous forme de solvate hémiéthanolate de formule 3, avantageusement sous vide.

En effet le traitement du phosphate libre de formule 2 en solution dans l'éthanol, anhydre et en présence d'ultrasons, avantageusement pendant 20 min, favorise l'apparition de la forme cristalline. Il se forme un solvate avec ½ molécule d'éthanol de cristallisation. Ce phénomène n'apparaît pas être général car des essais comparatifs avec le méthanol ont été effectués. Aucune cristallisation n'est alors apparue. De la même façon, des conditions équivalentes ont été appliquées aux différents composés de la famille du produit de formule 2, par exemple, les produits possédants une chaîne phénoxyacétate, 4-trifluorométhoxyphénoxyacétate, 4-méthylphénoxyacétate en lieu et place de la chaîne pentafluorophénoxyacétate, produits mentionnés dans la demande de brevet WO 96/12727. Aucune cristallisation n'intervient avec ces structures similaires. Seul le composé de formule 2 possédant la chaîne pentafluorophénoxyacétate a la particularité de former des cristaux par le traitement mentionné ci-dessus.

Après plusieurs recristallisations ce composé garde toujours la même stoechiométrie d'éthanol, soit ½ mole de solvant par mole du composé, même après un séchage sous vide à température ambiante. On obtient à ce stade un solvate, hémiéthanolate de formule 3.

En outre, il est apparu qu'un séchage plus poussé sous vide à 40°C pendant 10 jours permet alors d'éliminer difficilement mais totalement l'éthanol. Il est ainsi nécessaire de passer par une forme cristallisée en tant que solvate avant d'obtenir une forme cristallisée sans solvant. Ce composé ne peut pas être obtenu directement.

Il est bien entendu que la difficulté majeure de la cristallisation réside dans l'obtention du premier cristal, et que d'autres techniques de cristallisation peuvent être mises en jeu et aboutir à une cristallisation de lots futurs par ensemencement avec des cristaux déjà formés. Ainsi, la présente invention concerne en outre un procédé de préparation du composé cristallin du 4'-déméthyl-4'-phosphate-2",3"-bispentafluorophénoxyacétyl-4",6"-éthylidène-β-D-glucoside d'épipodophyllotoxine sous forme de solvate avec l'éthanol caractérisé en ce qu'il comprend les étapes successives suivantes :
a) ajout d'éthanol au composé 2 sous forme amorphe,
b) concentration de la solution,
c) cristallisation du 4'-déméthyl-4'-phosphate-2",3"-bispentafluorophénoxyacétyl-4",6"-éthylidène-β-D-glucoside d'épipodophyllotoxine sous forme de solvate avec l'éthanol par refroidissement à 10°C pendant 1,5 h de la solution concentrée et ajout d'une amorce constituée par un cristal du composé de formule 2 sous forme libre ou du composé de formule 3 sous forme de solvate hémiéthanolate,
d) filtration à 10°C et rinçage à l'éthanol,
e) séchage du produit obtenu.
L'étape (a) de concentration de la solution est avantageusement réalisée par évaporation d'environ 50% de la solution.
Avantageusement, si le produit cristallisé obtenu par le procédé selon la présente invention n'est pas conforme, une deuxième recristallisation est réalisée en resolubilisant dans l'acétone, en préconcentrant la solution obtenue, avantageusement en évaporant 75 % de la solution, puis en reprenant les étapes (a) à (e).

Avantageusement, l'étape (e) de séchage du produit est réalisée à l'étuve, sous vide et à 40°C.

Il est en outre apparu qu'un séchage poussé sous vide (avantageusement à 40°C pendant 10 jours) permet alors d'éliminer difficilement mais totalement l'éthanol. On obtient ainsi une forme cristallisée sans solvant, c'est-à-dire le composé cristallin de formule 2 sous forme libre.

La présente demande concerne également un médicament comprenant un composé cristallin selon la présente demande (avantageusement de formule 2 ou 3).
Avantageusement ce médicament est destiné au traitement anticancéreux avantageusement des tumeurs liquides ou des tumeurs solides, en particulier, le cancer du poumon à petites cellules, les tumeurs embryonnaires, les neuroblastomes, le cancer du rein, les tumeurs pédiatriques, les lymphomes hodgkimiens et non hodgkimiens, les leucémies aigües, les choriocarcinomes placentaires, les adénocarcinomes mammaires.
En particulier, ce médicament est destiné à augmenter l'efficacité thérapeutique des composés inhibiteurs des topoisomérases 1 ou 2 pour le traitement des tumeurs réfractaires aux thérapies usuelles telles que les cancers colorectaux, mélanomes, gliomes, cancer de la prostate et du sein.
Dans un mode de réalisation avantageux, le présent médicament est un anti-tumoral destiné à traiter tout type de tumeur, que ce soit des tumeurs liquides ou des tumeurs solides.
De façon avantageuse, le médicament selon la présente invention se trouve sous une forme destinée à une administration orale, avantageusement sous forme de gélules, de capsules, de comprimés, ou parentérale, avantageusement à la posologie par 24h de 5 à 400 mg/m² pour la voie orale et de 2 à 200 mg/m² pour la voie injectable.
Le Médicament selon la présente invention peut être administré à des patients en première ligne ou après un ou plusieurs traitements par chirurgie, radiothérapie ou chimiothérapie. Il peut être administré seul ou en combinaison avec d'autres chimiothérapies. Ce médicament peut donc en outre comprendre un autre anticancéreux.
Les exemples suivants sont donnés à titre indicatif non limitatifs

### PROCEDE DE FABRICATION

### Exemple 1 :

### Préparation du ½ éthanolate de 4'-déméthyl-4'-phosphate-2",3"-bispentafluorophénoxyacétyl-4",6"-éthylidène-β-D-glucoside d'épipodophyllotoxine à partir du 4'-déméthyl-4'-dibenzylphosphate-2",3"-bispentafluorophénoxyacétyl-4",6"-éthylidène-(-D-glucoside d'épipodophyllotoxine sous forme amorphe.

Le phosphate libre de formule 2 sous forme amorphe est obtenu soit par la méthode décrite dans la demande de brevet WO 96/12727 à l'exemple 22, soit selon celle décrite à l'exemple 8 du brevet FR 2 791 682.
Par exemple: 15g (11,6 mmol) de 4'-déméthyl-4'-dibenzylphosphate-2",3"-bispentafluorophénoxyacétyl-4",6"-éthylidène-(-D-glucoside d'épipodophyllotoxine sont placés à hydrogéner dans 200 ml d'un mélange acétate d'éthyle - éthanol (1/3), sous atmosphère d'hydrogène, à pression ordinaire, avec 1,2g de charbon palladié à 10%, pendant 30 min sous agitation. Le milieu réactionnel est filtré, le filtrat est évaporé et trituré dans l'éther isopropylique pour obtenir une poudre amorphe de formule 2.
Fusion (Electrothermal 9300 à capillaire) :~ 138 °C (ramollissement). Pureté analytique HPLC : 99,5 %. CCM : Rf 0,5, Solvant : Dichlorométhane - méthanol - acide acétique 90/10/5.
Diagramme de rayon X : figure 1

Préparation du ½ éthanolate :
10g de la forme amorphe obtenue ci-dessus sont dissous dans 500 ml d'éthanol. Cette solution est placée dans un bac à ultrasons (Bioblock-Scientific type T 700, fréquence 35 kHz) pendant 20 min, à 30°C. Un précipité épais apparaît qui est ensuite filtré et séché sous vide. On obtient 7 g de cristaux.
Fusion : 161°C Le spectre RMN montre la présence de ½ mole d'éthanol. Ces cristaux sont recristallisés une deuxième fois puis une troisième fois dans 130 ml d'éthanol au reflux, puis laissé à refroidir lentement 12 h. On obtient après filtration et séchage sous vide des cristaux.
Fusion 165-166°C. Le spectre de RMN montre à nouveau la présence de ½ mole d'éthanol.
Diagramme de rayon X : figure 3

### Exemple 2:

### Préparation du 4'-déméthyl-4'-phosphate-2",3"-bis pentafluorophénoxyacétyl-4",6"-éthylidène-β-D-glucoside d'épipodophyllotoxine sous forme cristalline à partir du ½ éthanolate de 4'-déméthyl-4'-phosphate-2",3"-bis pentafluorophénoxyacétyl-4",6"-éthylidène-β-D-glucoside d'épipodophyllotoxine sous forme cristalline

Une aliquote des cristaux obtenus ci-dessus est placée, en couche fine, dans un dessiccateur chauffant à 40°C pendant 10 jours. Le spectre de RMN montre la perte d'éthanol.
Fusion 169°C.
Diagramme de rayon X : figure 2

### Exemple 3 :

### Préparation d'un solvate cristallin avec l'éthanol du 4'-déméthyl-4'-phosphate-2",3"-bis pentafluorophénoxyacétyl-4",6"-éthylidène-β-D-glucoside d'épipodophyllotoxine autre que le hémiéthanolate

Le phosphate de formule 2 sous forme amorphe est obtenu soit par la méthode décrite dans la demande de brevet WO 96/12727 à l'exemple 22, soit selon celle décrite à l'exemple 8 du brevet FR 2 791 682.

Les étapes de cristallisation sont ensuite les suivantes:
- ajout d'EtOH: environ un volume par rapport au volume initial avant concentration,
- concentration de la solution: évaporation d'environ 50% de la solution,
- refroidissement de la solution à 10°C et maintien de cette solution à 10° C durant 1,5 heures tout en ensemençant avec un crystal du composé obtenu selon l'exemple 1 ou 2.
- filtration à 10°C et rinçage à l'éthanol du produit cristallisé obtenu. (si le produit n'est pas conforme, une deuxième recistallisation est réalisée en resolubilisant dans l'acétone et en préconcentrant, puis en reprenant les étapes indiquées ci-dessus),
- séchage du produit à l'étuve: sous vide et à 40°C.
On obtient un solvate cristallin avec l'éthanol
Diagramme de rayon X : figures 4 et 5
Pureté HPLC : 99,1 %
Teneur en éthanol : 1,4 %
Teneur en eau : inférieure à 1 %.

### Exemple 4:

### Préparation du 4'-déméthyl-4'-phosphate-2",3"-bis pentafluorophénoxyacétyl-4",6"-éthylidène-β-D-glucoside d'épipodophyllotoxine sous forme cristalline à partir du solvate avec l'éthanol du 4'-déméthyl-4'-phosphate-2",3"-bis pentafluorophénoxyacétyl-4",6"-éthylidène-β-D-glucoside d'épipodophyllotoxine sous forme cristalline (exemple 3).

12 g des cristaux obtenus ci-dessus (exemple 3) sont placés dans un large cristallisoir à l'intérieur d'une cloche à vide à plateau chauffant. Le vide est installé et la température fixée à 40°C.
Les cristaux sont séchés pendant 10 jours. Au terme de ces 10 jours, l'éthanol est totalement évaporé.
Un diffractogramme de Rayons X a été effectué montrant la parfaite cristallinité (figure 6).

### Exemple 5:

### Essais de cristallisation du 4'-déméthyl-4'-phosphate-2",3"-bisphénoxyacétyl-4",6"-éthylidène-β-D-glucoside d'épipodophyllotoxine, du 4'-déméthyl-4'-phosphate-2",3"-bis(4-trifluorométhoxyphénoxyacétyl)-4",6"-éthylidène-β-D-glucoside d'épipodophyllotoxine et du 4'déméthyl-4'-phosphate-2",3"-bis(4-méthylphénoxyacétyl)-4",6"-éthylidène-β-D-glucoside d'épipodophyllotoxine.

Les 4'-déméthyl-4'-phosphate-2",3"-bisphénoxyacétyl-4",6"-éthylidène-(-D-glucoside d'épipodophyllotoxine, 4'-déméthyl-4'-phosphate-2",3"-bis(4-trifluorométhoxyphénoxyacétyl)-4",6"-éthylidène-(-D-glucoside d'épipodophyllotoxine, et 4'déméthyl-4'-phosphate-2",3"-bis(4-méthylphénoxyacétyl)-4",6"-éthylidène-(-D-glucoside d'épipodophyllotoxine sous forme amorphe sont obtenus selon le procédé décrit dans la demande de brevet WO 96/12727.
100 mg de ces composés sont dissous dans 5 ml d'éthanol à température ambiante. Ces différentes solutions sont placées dans le bac à ultrasons pendant 20 min. Aucune cristallisation n'apparaît même après trituration des parois, ou en laissant au repos plusieurs jours, selon les méthodes usuelles pour amorcer la cristallisation.

### ETUDE DE LA FORME CRISTALLINE

Des études de diffraction de rayons X sur les différentes formes obtenues ci-dessus (formule 2 sous forme amorphe, sous forme cristalline, formule 3 sous forme cristalline et autre composé cristallin sous forme de solvate avec l'éthanol) ainsi que de thermomicroscopie ont été effectuées sur plusieurs échantillons pour la mise en évidence de l'existence de la forme cristalline.

### Diffractions des Rayons X :

Le composé de formule 2 sous forme amorphe et sous forme cristalline, le composé de formule 3 hémiéthanolate et un solvate cristallin avec l'éthanol du composé 4'-déméthyl-4'-phosphate-2",3"-bispentafluorophénoxyacétyl-4",6"-éthylidène-β-D-glucoside d'épipodophyllotoxine autre que l'hémiéthanolate obtenu ci-dessus ont été étudiés par analyse en diffraction des rayons X sur poudre.
L'appareillage est un Philips PN 1730 équipé d'un goniomètre CGR type C horizontal anti-cathode cuivre (λ= 1,54051 Angström, 1 = 20 mA et V = 40 kV) (figures 1 à 4).
Les échantillons sont soumis à l'analyse dans les conditions opératoires suivantes :
- Domaine d'angle exploré de 3 à 25 degré θ
- Temps d'acquisition par point de 500 ms
- 5 acquisitions (figures 1 à 4).

Pour la figure 5, l'appareillage est un Philips X'Pert PW 3040 équipé d'un goniomètre avec anti-cathode cuivre (raie Kα).
Le domaine d'angle exploré est 2 à 50 °2θ.
Le comptage est de 2 ou 2,4s par pas, le pas ayant une valeur de 0,02 °2θ.
L'échantillon a été mesuré sans traitement spécial autre que l'application d'une faible pression pour obtenir une surface plane. Une feuille de Kapton a été placée sur l'échantillon pour éviter la contamination de la chambre par l'échantillon cytostatique. L'atmosphère utilisée est l'air ambiant.

Pour la figure 6, le diagramme a été enregistré sur un appareillage Philips X'Pert MPD équipé d'un détecteur Xcelerator et d'une anticathode cuivre (raie K alpha, intensité : 20mA, tension 40 KV, longueur d'onde lambda: 1,5418367 angström) sans monochromateur et avec filtre nickel.
L'enregistrement a été réalisé en mode continu. Les scans sont en oméga 2θ.
Le comptage est de 100s par pas, la valeur du pas étant de 0,02° 2θ.

L'intensité à 100 % est attribuée à la raie dont la valeur du nombre de coups est la plus importante. Le nombre de coups est proportionnel à l'intensité absolue de chacune des raies.

### Résultats :

Le diffractogramme montre que la cristallinité (liée au nombre de coups de la raie à 100 %) est nettement très importante dans le cas de l'échantillon de formule 2 (figures 2 et 6) et également dans celui de l'échantillon solvate de formule 3 (figure 3) et dans l'autre solvate (figures 4 et 5). L'échantillon amorphe de formule 2 ne présente pas de telles raies (figure 1).

### Thermomicroscopie :

Une étude physico-chimique de thermomicroscopie a été effectuée montrant un état cristallin avec l'hémiéthanolate de formule 3 par montée en température.
L'appareillage utilisé est le système FP52 relié au programmateur FP5 (Mettler). Les échantillons sont placés entre lame et lamelle et chauffés selon une vitesse programmée entre 30 et 150°C.
L'échantillon de formule 2 sous forme amorphe reste amorphe et ne cristallise pas en montée de température.
L'échantillon hémiéthanolate de formule 3 est cristallin. Il présente une perte de solvant jusqu'à la température de 150°C, la fusion intervient vers 165°C avec décomposition.

### ETUDE DE STABILITE

### Mise en évidence de la stabilité en poudre.

Des études de stabilité en poudre on été réalisées sur le composé de formule 2 sous forme amorphe et sur le composé de formule 3 sous forme cristalline obtenus ci-dessus, dans les conditions suivantes :
A 40°C en flacon fermé pendant 1 mois.
A 30°C, à 75 % d'humidité relative, en flacon ouvert pendant 1 mois.
Pour les études de stabilité en flacon fermé, les échantillons sont scellés dans des fioles en verre ambré (Alltech ref. vial 98037, ref. bouchon 6687).
Les études de stabilité en flacon ouvert sont réalisées par stockage de la poudre dans un dessiccateur contenant une solution saline, saturée de NaCl (75% d'humidité relative /30°C).
La teneur en composé phosphate libre de formule 2 est évaluée par normalisation interne avec le système chromatographique HPLC suivant :
   Colonne : Symmetry C₈, 5 *µ*, 250 x 4,6 mm (Waters)
   Eluant : CH₃CN / H₂O / KH₂PO₄ 600 / 400 / 3,4 (ml/ml/g) ajusté à pH 4 avec H₃PO₄ à 10%
   Débit : 1 ml/min
   Détection : 220 nm
   Les analyses sont effectuées sur la chaîne HPLC Lachrom-Merck Hitachi équipée d'une pompe L-7100, d'un passeur d'échantillon L-7200, d'un détecteur à barette de diodes L-7450 et d'un logiciel de traitement HSM D-7000.

Les résultats de cette étude sont résumés dans le tableau 1 suivant:

**Tableau 1 : % de produits de dégradation du composé de formule 2 sous forme amorphe et du composé de formule 3 sous forme cristalline selon les conditions de conservation**

| | **Conditions de conservation** | |
|---|---|---|
| **Composés** | **% de produits de dégradation après 1 mois à 40°C en flacon fermé** | **% de produits de dégradation après 1 mois à 30°C avec 75 % d'humidité relative** |
| **Composé de formule 2 sous forme amorphe** | 11,9 % | 50 % |
| **Composé de formule 3 sous forme cristalline** | 1,5% | 1,8% |

Les études de stabilité en poudre montrent que le composé de formule 2 sous forme amorphe est moins stable que le composé hémiéthanolate de formule 3 sous forme cristalline quelles que soient les conditions de stabilité :
A 40°C après 1 mois en flacon fermé hermétiquement, 11,9 % de produits de dégradation sont apparus pour le composé de formule 2 sous forme amorphe, contre 1,5 % pour le composé hémiéthanolate de formule 3 sous forme cristalline.
A 30°C après 1 mois en présence d'humidité relative, 50 % de produits de dégradation sont apparus pour le composé de formule 2 sous forme amorphe, contre 1,8 % pour le composé hémiéthanolate de formule 3 sous forme cristalline.
Une caractéristique de l'invention est ainsi l'avantage en terme de stabilité de la forme cristalline hémiéthanolate de formule 3 par rapport à sa forme libre amorphe (formule 2).

### ETUDE PHARMACOLOGIQUE

L'utilisation au stade des études cliniques de la forme de phosphate salifiée avec la N-méthyl-D-glucamine de formule 1 ne pouvant pas s'effectuer dans de bonnes conditions, l'étude de la forme phosphate libre amorphe de formule 2 a été envisagée. Il est apparu au cours de l'étude pharmacologique que ce composé sous sa forme phosphate libre amorphe ne possédait pas l'activité in vivo mise en évidence avec son sel de N-Méthyl-D-Glucamine, tel que cela a été publié dans *British Journal of Cancer* (2000), 83(11), 1516-1524. Par contre, la forme cristalline obtenue soit sous forme de hémiéthanolate de formule 3 soit sous forme libre de formule 2 présente l'activité pharmacologique requise.

### Matériel et méthodes

### Mise en solution des composés pour l'évaluation in vivo.

Les différentes formes amorphes et cristallines des composés de formule 2 et 3 sont mises en solution/suspension dans un mélange contenant 5% de tween 80 et 95% de sérum glucosé à 5%.

### Modèle de tumeur expérimentale.

Le modèle utilisé est la leucémie P388 (Dykes, D.J. and Waud, W.R. : Murine L1210 and P388 Leukemias. *In* Tumor Models in Cancer Research. Teisher, B.A. ed., Humana Press Inc., Totowa, NJ. pp. 23-40, 2002), qui est maintenue par transplantations intra péritonéales successives sur des souris DBA/2 (DBA/2JIco, Charles River) ainsi qu'il a été décrit précédemment (Kruczynski, A. and Hill, B.T. : Classic *in vivo* cancer models : Three examples of mouse models used in expérimental therapeutics. Current Protocols in Pharmacology Unit 5.24 : 5.24.1-5.24.16, 2001).

### Chimiothérapie expérimentale

Les animaux sont hébergés, entretenus et manipulés en accord avec le Guide de Soins et d'Utilisation des Animaux de Laboratoire (National Research Council, 1996) et avec la Directive Européenne EEC/86/609, sous la direction des chercheurs autorisés à encadrer l'expérimentation animale. De plus, toutes les expériences sont conduites conformément à la législation française et aux règles du Comite d'Ethique du Centre de Recherche Pierre Fabre, qui sont basées sur les directives de l'UKCCCR (United Kingdom Coordinating Committee on Cancer Research) pour les soins aux animaux en oncologie expérimentale (Workman, P., Twentyman, P., Balkwill, F., Balmain, A., Chaplin, D., Double, J., Embleton, J., Newell, D., Raymond, R., Stables, J., Stephens, T., and Wallace, J., : United Kingdom co-ordinating committee on cancer research (UKCCCR) guidelines for the walfare of animals in expérimental neoplasia. Br J. Cancer 77 : 1-10, 1998). L'expérience est réalisée selon un protocole déjà décrit précédemment (Kruczynski, A., Colpaert, F., Tarayre, J.P., Mouillard, P., Fahy, J., and Hill, B.T. : Preclinical *in vivo* antitumor activity of vinflumine, a novel fluorinated *Vinca* alkaloid. Cancer Chemother. Pharmacol. 41 : 437-447, 1998). Cela consiste à implanter 10⁶ cellules de leucémie P388 par souris aux souris hybrides C2DF1 (CD2F1/Cr1BR, Charles River, St Aubin-les-Elbeuf, France) par voie intraveineuse au jour zéro. Après randomisation des animaux dans les cages de traitement et contrôle, les composés à évaluer sont administrés en injection unique par voie intra péritonéale le lendemain de la greffe tumorale, au jour 1. Les animaux sont ensuite surveillés tous les jours, pesés deux fois par semaine et toute réaction clinique notée.

### Evaluation de l'activité anti-tumorale

La survie est le paramètre d'évaluation de l'activité anti-tumorale. L'augmentation de survie est définie par le rapport T/C (%), correspondant à : (Médiane de survie du groupe traité / Médiane de survie du groupe de contrôle) x 100.

### Résultats

### Evaluation de l'activité anti-tumorale des différentes formes des composés de formule 2 et 3 sur le modèle de la leucémie P388 in vivo.

Les trois formes de composés ont été évaluées *in vivo,* soient : le composé de formule 2 sous forme amorphe (formule 2 amorphe), le composé de formule 2 sous forme cristalline, séchée afin d'éliminer toute trace du solvant éthanol, (formule 2 cristalline) et le composé de formule 3 sous forme cristalline contenant une demi-mole d'éthanol (formule 3 cristalline). Les résultats sont rassemblés dans le tableau 2 suivant :

**Tableau 2 : valeur de T/C (en %) pour les composés de formule 2 ou 3 sous forme amorphe ou cristalline en fonction de la dose de composé injectée**

| ***Valeurs des T*/*C (%):*** | **Dose (mg/kg)** | | |
|---|---|---|---|
| **Composé** | **40** | **80** | **160** |
| Formule 2 cristalline | 143 | 157 | 300 |
| Formule 3 cristalline | 143 | 143 | 329 |
| Formule 2 amorphe | 100 | 114 | 157 |

Les résultats montrent que les deux composés de formule 2 et 3 sous forme cristalline induisent une augmentation de survie des animaux significative dès la dose de 40mg/kg, ce qui est reflété par une valeur du ratio T/C de 143 %, indiquant que le traitement des animaux par l'un ou l'autre des deux composés sous forme cristalline a permis de prolonger la survie des animaux de 43 %. En effet, selon les critères du NCI (National Cancer Institute), une valeur de T/C est considérée significative si elle est au moins supérieure à 120 % et elle est considérée très hautement significative ou reflétant une activité anti-tumorale de haut niveau si elle est supérieure à 175 % (Venditti, J.M. : Preclinical drug development : Rationale and methods. Semin. Oncol. 8 : 349-361, 1981). L'activité anti-tumorale induite par les composés de formule 2 ou 3 sous forme cristalline augmente avec la dose de composé pour atteindre une valeur optimale à la dose de 160mg/kg, ce qui est reflété par des valeurs de T/C de 329 % pour le composé de formule 3 sous forme cristalline et de 300 % pour le composé de formule 2 sous forme cristalline. Ces valeurs indiquent que le traitement des animaux porteurs de leucémie P388 par les composés de formule 2 ou 3 sous forme cristalline a permis de prolonger la survie des animaux de 200 % ou 229 % respectivement.
Contrairement aux formes cristallines, le composé de formule 2 sous forme amorphe n'induit pas d'activité anti-tumorale significative aux doses de 40 et 80 mg/kg, ce qui est reflété par des valeurs de T/C inférieures à 120 %, soient 100 % et 114 %, respectivement. Le composé de formule 2 sous forme amorphe induit une activité anti-tumorale seulement à la dose de 160 mg/kg mais qui est cependant très inférieure à l'activité obtenue avec les composés de formule 2 ou 3 sous forme cristalline à la même dose. En effet, la valeur de T/C obtenue à la dose de 160 mg/kg pour le composé de formule 2 sous forme amorphe est de 157 %, alors qu'elle est de 329 % et 300 % respectivement pour les composés de formules 3 et 2 sous forme cristalline.
Il est à noter que ces activités n'induisent aucune perte de poids significative des animaux, suggérant que les composés sont bien tolérés par l'animal.

En conclusion, les composés de formules 2 ou 3 sous forme cristalline induisent une activité anti-tumorale de haut niveau sur le modèle de la leucémie P388 et qui est nettement supérieure à l'activité du composé de formule 2 sous forme amorphe.
Ces composés présentent donc un intérêt majeur dans l'arsenal thérapeutique anticancéreux. Par administration parentérale, en perfusion, ou par voie orale, ils permettent de soigner tous types de cancers, que ce soit des tumeurs liquides ou des tumeurs solides. Ils peuvent administrés à des patients en première ligne ou après un ou plusieurs traitements par chirurgie, radiothérapie ou chimiothérapie. Ces composés peuvent être administrés seul ou en combinaison avec d'autres chimiothérapies.

## Revendications

1. Composé cristallin 4'-déméthyl-4'-phosphate-2",3"-bispentafluorophénoxyacétyl-4",6"-éthylidène-β-D-glucoside d'épipodophyllotoxine sous forme libre de formule 2 suivante : 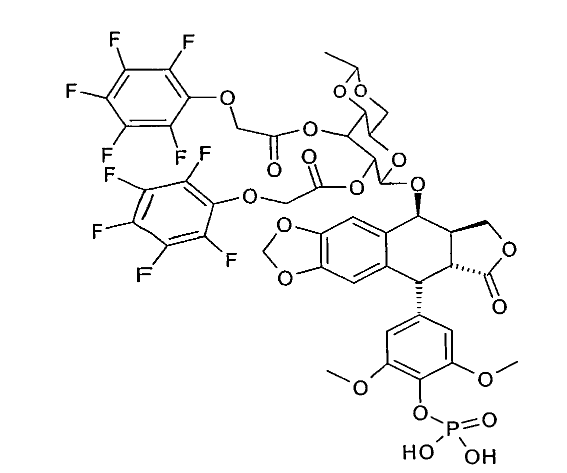 ou sous forme de solvate avec l'éthanol.

2. Composé cristallin selon la revendication 1 **caractérisé en ce que** le solvate avec l'éthanol est un solvate hémiéthanolate de formule 3 suivante :

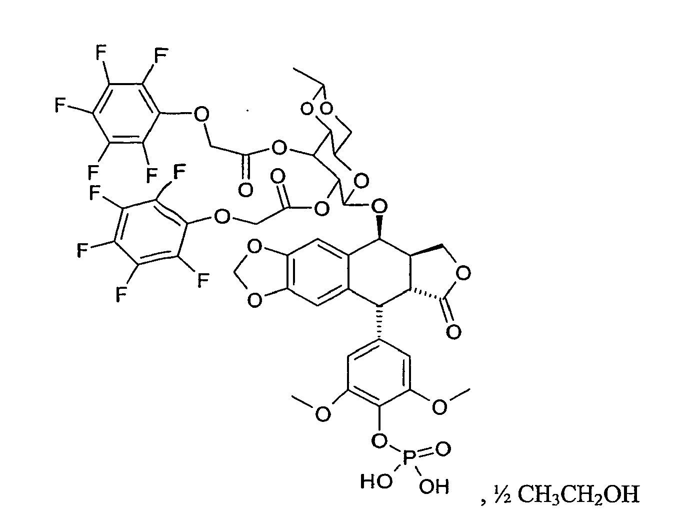

3. Composé cristallin selon la revendication 1 sous forme libre de formule 2 **caractérisé en ce que** son diagramme de rayon X correspond sensiblement à celui des figures 2 ou 6.

4. Composé cristallin selon les revendications 1 ou 2 sous forme de solvate hémiéthanolate de formule 3 **caractérisé en ce que** son diagramme de rayon X correspond sensiblement à celui de la figure 3.

5. Procédé de préparation du composé cristallin selon les revendications 1, 2 ou 4 sous forme de solvate hémiéthanolate **caractérisé en ce que** le composé de formule 2 sous forme amorphe est mis en solution dans l'éthanol anhydre en présence d'ultrasons.

6. Procédé selon la revendication 5 **caractérisé en ce que** les ultrasons sont appliqués pendant au moins 20 minutes.

7. Procédé de préparation du composé cristallin selon les revendications 1 ou 3 sous forme libre **caractérisé en ce que** le composé cristallin sous forme de solvate hémiéthanolate de formule 3 est séché, avantageusement sous vide.

8. Procédé de préparation du composé cristallin selon les revendications 1, 2 ou 4 sous forme de solvate avec l'éthanol **caractérisé en ce qu'**il comprend les étapes successives suivantes :
a) ajout d'éthanol au composé de formule 2 sous forme amorphe,
b) concentration de la solution,
c) cristallisation du 4'-déméthyl-4'-phosphate-2",3"-bispentafluorophénoxyacétyl-4",6"-éthylidène-β-D-glucoside d'épipodophyllotoxine sous forme de solvate avec l'éthanol par refroidissement à 10°C pendant 1,5 h de la solution concentrée et par ajout d'une amorce constituée par un cristal du composé de formule 2 sous forme libre ou du composé de formule 3 sous forme de solvate hémiéthanolate,
d) filtration à 10°C et rinçage à l'éthanol,
e) séchage du produit obtenu.

9. Médicament comprenant un composé cristallin selon l'une quelconque des revendications 1 à 4.

10. Médicament selon la revendication 9 destiné au traitement anticancéreux des tumeurs liquides ou des tumeurs solides, en particulier, le cancer du poumon à petites cellules, les tumeurs embryonnaires, les neuroblastomes, le cancer du rein, les tumeurs pédiatriques, les lymphomes hodgkimiens et non hodgkimiens, les leucémies aigües, les choriocarcinomes placentaires, les adénocarcinomes mammaires

11. Médicament selon la revendication 9 destiné à augmenter l'efficacité thérapeutique des composés inhibiteurs des topoisomérases 1 et 2, pour le traitement des tumeurs réfractaires aux thérapies usuelles telles que les cancers colorectaux, les mélanomes, les gliomes, les cancers de la prostate et du sein.

12. Médicament selon l'une quelconque des revendications 9 à 11 **caractérisé en ce qu'**il est sous une forme destinée à une administration orale ou parentérale.

13. Médicament selon l'une quelconque des revendications 9 à 11 **caractérisé en ce qu'**il comprend en outre un autre anticancéreux.

## Claims

1. A crystalline compound 4'-demethylepipodophyllotoxin 2",3"-bispentafluorophenoxyacetyl-4",6"-ethylidene-β-D-glucoside 4'-phosphate in free form of formula 2 below: 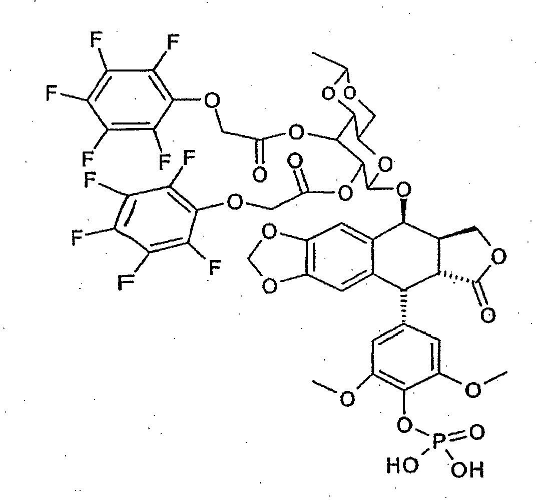 or in the form solvated with ethanol.

2. The crystalline compound as claimed in claim 1, **characterized in that** the solvate with ethanol is a hemiethanolate solvate of formula 3 below:

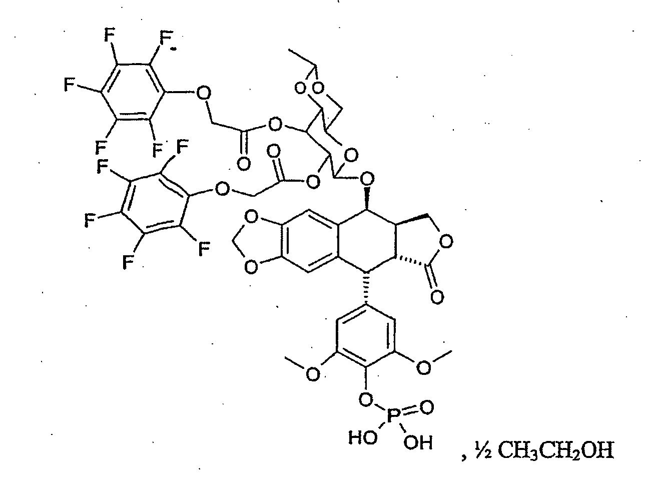

3. The crystalline compound as claimed in claim 1 in free form of formula 2, **characterized in that** its X-ray diagram corresponds substantially to that of figure 2 or 6.

4. The crystalline compound as claimed in claim 1 or 2 in the form of the hemiethanolate solvate of formula 3, **characterized in that** its X-ray diagram corresponds substantially to that of figure 3.

5. A method for preparing the crystalline compound as claimed in claim 1, 2 or 4 in the form of the hemiethanolate solvate, **characterized in that** the compound of formula 2 in amorphous form is dissolved in anhydrous ethanol in the presence of ultrasound.

6. The method as claimed in claim 5, **characterized in that** the ultrasound is applied for at least 20 minutes.

7. A method for preparing the crystalline compound as claimed in claim 1 or 3 in free form, **characterized in that** the crystalline compound in the form of the hemiethanolate solvate of formula 3 is dried, advantageously under vacuum.

8. A method for preparing the crystalline compound as claimed in claim 1, 2 or 4 in the form solvated with ethanol, **characterized in that** it comprises the following successive steps:
a) addition of ethanol to the compound of formula 2 in amorphous form,
b) concentration of the solution,
c) crystallization of the 4'-demethylepipodophyllotoxin 2",3"-bispentafluorophenoxyacetyl-4",6"-ethylidene-β-D-glucoside 4'-phosphate in the form solvated with ethanol, by cooling the concentrated solution at 10°C for 1.5 h and adding an initiator consisting of a crystal of the compound of formula 2 in free form or of the compound of formula 3 in the form of the hemiethanolate solvate,
d) filtration at 10°C and rinsing with ethanol,
e) drying of the product obtained.

9. A medicinal product comprising a crystalline compound as claimed in any one of claims 1 to 4.

10. The medicinal product as claimed in claim 9, for use in anticancer treatment for liquid tumors or solid tumors, in particular small cell lung cancer, embryonic tumors, neuroblastomas, kidney cancer, pediatric tumors, Hodgkins and nonhodgkins lymphomas, acute leukemias, placental choriocarcinomas, and mammary adenocarcinomas.

11. The medicinal product as claimed in claim 9, intended to increase the therapeutic efficacy of topoisomerase 1 and 2 inhibitor compounds, for the treatment of tumors that are refractory to the usual therapies, such as colorectal cancers, melanomas, gliomas, prostate cancer and breast cancer.

12. The medicinal product as claimed in any one of claims 9 to 11, **characterized in that** it is in a form intended for oral or parenteral administration.

13. The medicinal product as claimed in any one of claims 9 to 11, **characterized in that** it also comprises another anticancer agent.

## Patentansprüche

1. Die kristalline Verbindung 4'-Demethyl-4'-phosphat-2",3"-bispentafluorphenoxyacetyl-4",6"-ethyliden-β-D-glucosid von Epipodophyllotoxin in freier Form der folgenden Formel 2: 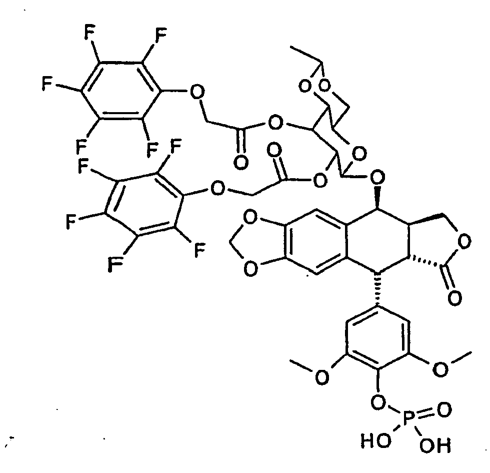 oder in Form eines Solvats mit Ethanol.

2. Kristalline Verbindung nach Anspruch 1, **dadurch gekennzeichnet, dass** das Solvat mit Ethanol ein Hemiethanolat-Solvat der folgenden Formel 3 ist:

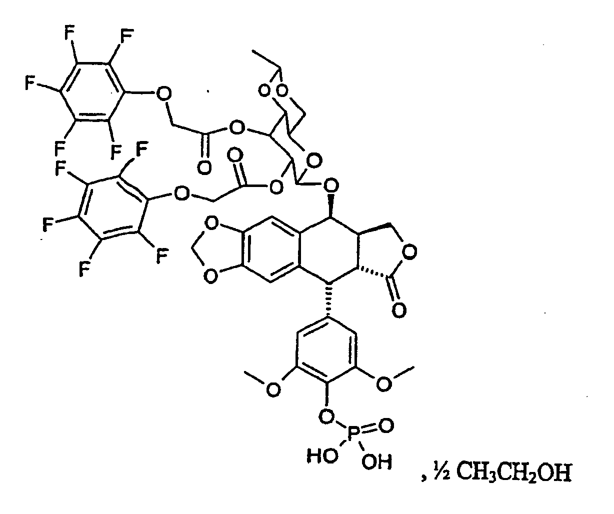

3. Kristalline Verbindung nach Anspruch 1 in freier Form der Formel 2, **dadurch gekennzeichnet, dass** ihr Röntgendiagramm ungefähr jenem der Figur 2 oder 6 entspricht.

4. Kristalline Verbindung nach den Ansprüchen 1 oder 2 in Form des Hemiethanolat-Solvats der Formel 3, **dadurch gekennzeichnet, dass** ihr Röntgendiagramm ungefähr jenem der Figur 3 entspricht.

5. Verfahren zur Herstellung der kristallinen Verbindung nach den Ansprüchen 1, 2 oder 4 in Form des Hemiethanolat-Solvats, **dadurch gekennzeichnet, dass** die Verbindung der Formel 2 in amorpher Form in wasserfreiem Ethanol in Anwesenheit von Ultraschall gelöst wird.

6. Verfahren nach Anspruch 5, **dadurch gekennzeichnet, dass** der Ultraschall während mindestens 20 Minuten angewendet wird.

7. Verfahren zur Herstellung der kristallinen Verbindung nach den Ansprüchen 1 oder 3 in freier Form, **dadurch gekennzeichnet, dass** die kristalline Verbindung in Form des Hemiethanolat-Solvats der Formel 3 getrocknet wird, vorteilhaft unter Vakuum.

8. Verfahren zur Herstellung der kristallinen Verbindung nach den Ansprüchen 1, 2 oder 4 in Form eines Solvats mit Ethanol, **dadurch gekennzeichnet, dass** es die folgenden aufeinanderfolgenden Schritte umfasst:
a) Zugabe von Ethanol zur Verbindung der Formel 2 in amorpher Form,
b) Konzentrieren der Lösung,
c) Kristallisation des 4'-Demethyl-4'-phosphat-2",3"-bispentafluorphenoxyacetyl-4",6"-ethyliden-β-D-glucosids von Epipodophyllotoxin in Form eines Solvats mit Ethanol durch Kühlen der konzentrierten Lösung auf 10°C während 1,5 h und durch Zugabe eines Keimkristalls, der aus einem Kristall der Verbindung der Formel 2 in freier Form oder der Verbindung der Formel 3 in Form des Hemiethanolat-Solvats besteht,
d) Filtration bei 10°C und Spülen mit Ethanol,
e) Trocknen des erhaltenen Produkts.

9. Medikament, umfassend eine kristalline Verbindung nach irgendeinem der Ansprüche 1 bis 4.

10. Medikament nach Anspruch 9, das bestimmt ist zur Antikrebs-Behandlung von Flüssigkeitstumoren oder festen Tumoren, insbesondere von kleinzelligem Lungenkrebs, embryonalen Tumoren, Neuroblastomen, Nierenkrebs, pädiatrischen Tumoren, Hodgkin-Lymphom und non-Hodgkin-Lymphom, akuten Leukämien, Plazenta-Chorioblastomen, Mama-Adenokarzinomen.

11. Medikament nach Anspruch 9, das zur Steigerung der therapeutischen Wirksamkeit von Inhibitor-Verbindungen der Topoisomerasen 1 und 2 und für die Behandlung von Tumoren bestimmt ist, die gegenüber üblichen Therapien refraktär sind, wie kolorektalen Krebsen, Melanomen, Gliomen, Prostata- und Brustkrebs.

12. Medikament nach irgendeinem der Ansprüche 9 bis 11, **dadurch gekennzeichnet, dass** es in einer Form vorliegt, die für eine orale oder parenterale Verabreichung bestimmt ist.

13. Medikament nach irgendeinem der Ansprüche 9 bis 11, **dadurch gekennzeichnet, dass** es darüber hinaus ein weiteres Antikrebsmittel umfasst.
